# EUROPEAN PATENT APPLICATION

(11) **EP 4 556 018 A1**
(43) Date of publication of application: **21.05.2025**
(21) Application number: 23839642.8
(22) Date of filing: 12.07.2023
(51) Int. Cl.: A61K 38/17, A61K 31/7088, A61K 35/17, A61K 45/00, A61K 48/00, A61P 35/00, C12N 5/10, C12N 15/09, C12N 15/113, G01N 33/50, G01N 33/53

(54) **AGENT FOR PREVENTING AND/OR TREATING TUMOR**

(30) Priority: 13.07.2022 JP 2022112660
(71) Applicant: OSAKA UNIVERSITY, Suita-shi Osaka 565-0871 (JP); Otsuka Pharmaceutical Co., Ltd., Tokyo 101-8535 (JP)
(72) Inventor: ICHIYAMA, Kenji, Suita-shi, Osaka 565-0871 (JP); SAKAGUCHI, Shimon, Suita-shi, Osaka 565-0871 (JP); HORITA, Yuji, Osaka-shi, Osaka 540-0021 (JP); KINOSHITA, Takeshi, Osaka-shi, Osaka 540-0021 (JP)
(74) Representative: Hoffmann Eitle
(86) International application number: PCT/JP2023/025690
(87) International publication number: WO 2024/014470

(57) **Abstract**

New medicaments for the prevention and treatment of tumors are developed.

A composition for preventing and/or treating tumors, which comprises an Ikzf1ΔE5 protein that is a deletion form of Ikzf1 in which exon 5 in Ikwzf1 is partially or completely deleted, an Ikzf1ΔE5 gene-related substance, or the like, as well as a method for screening the same.

## Description

### TECHNICAL FIELD

The present application claims the priority under the Paris Convention with respect to the Japanese Patent Application No. 2022-112660 filed on July 13, 2022, which is incorporated herein by reference in its entirety.

The present invention relates to compositions for the prevention and/or treatment of tumors. In particular, the invention relates to compositions for the prevention and/or treatment of tumors, which comprise Ikzf1ΔE5 proteins or Ikzf1ΔE5 gene-related substances, and methods for screening them.

### BACKGROUND ART

In recent years, research and development have focused on cancer immunotherapy. For example, an anti-CTLA-4 antibody, the immune checkpoint inhibitor Ipilimumab, is a medicament that enhances antitumor immunity by removing regulatory T cells (Tregs).

IKAROS family zinc finger 1 (Ikzf1) is one of the five IKAROS family transcription factors found in mammals, and is also referred to simply as "IKAROS". Ikzf1 is a molecule expressed in fetal and adult hematopoietic systems, which is responsible for chromatin remodeling and acts as a regulator of lymphocyte differentiation. It has been reported that loss-of-function mutations and deletions in Ikzf1 are found in B-lymphoblastic leukemia (B-Acute lymphoblastic leukemia, B-ALL) and are associated with poor prognosis. It has been also reported that Ikzf1 mutation in germ cell lines are associated with autoimmune diseases (Non-Patent Document 1).

Non-Patent Document 2 describes that the examination of the generated mice which express the deletion form of Ikzf1 from which exon 5 is deleted specifically in B cells has revealed that Ikzf is important in the differentiation from preB cells and that B lymphoblastic leukemia (B-ALL) is developed from preB cells whose differentiation was inhibited. This suggests that exon 5 in Ikzf1 should be important in B cell differentiation and B-ALL development. In addition, Patent Document 1 describes compositions and methods for inhibiting the Ikzf1 expression in a cell in order to enhance the cytolytic activity of the cell.

### CITATION LIST

### PATENT DOCUMENTS

Patent Document 1: WO2014/138348

### NON-PATENT DOCUMENTS

Non-Patent Document 1: Journal of Allergy and Clinical Immunology Volume 140, Issue 1, Pages 223-231 (2017)
Non-Patent Document 2: Nat Immunol. 2014 Mar; 15(3): 294-304. doi: 10. 1038/ni. 2821. Epub 2014 Feb 9

### SUMMARY OF THE INVENTION

### PROBLEMS TO BE SOLVED BY THE INVENTION

The purposes of the present invention is to develop new medicaments for the prevention and treatment of tumors.

### MEANS TO SOLVE PROBLEMS

The present inventors have earnestly studied aiming at developing a new method of prevention or treatment for immunotherapy, and, as a result, have found that, when a deletion form of Ikzf1 in which exon 5 is partially or completely deleted is expressed in a regulatory T cell (Treg cell) (hereinafter the deletion form may be referred to as "Ikzf1ΔE5". Also, a gene encoding Ikzf1ΔE5 may be referred to as "Ikzf1ΔE5 gene", and a protein encoded by the Ikzf1ΔE5 gene may be referred to as "Ikzf1ΔE5 protein".), the production of interferon gamma from the regulatory T cells is markedly enhanced and prevention and/or treatment for tumors are possible. Based on these findings, the inventors further investigated to complete the present invention.

Specifically, the present invention encompasses the aspects as follows.

### <Compositions for preventing and/or treating tumors: Part 1>

[1] A composition for preventing and/or treating tumors, which comprises an Ikzf1ΔE5 protein or an Ikzf1ΔE5 gene-related substance.
[2] The composition for preventing and/or treating tumors according to [1], wherein the composition targets a T-cell.
[3] The composition for preventing and/or treating tumors according to [2], wherein the T-cell is a CD4-positive T-cell.
[4] The composition for preventing and/or treating tumors according to [2], wherein the T-cell is a regulatory T-cell and/or a naive T-cell.
[5] The composition for preventing and/or treating tumors according to any one of [1] to [4], wherein the Ikzf1ΔE5 gene-related substance is a nucleic acid molecule encoding the Ikzf1ΔE5 protein.
[6] The composition for preventing and/or treating tumors according to any one of [1] to [4], wherein the Ikzf1ΔE5 gene-related substance is a substance that deletes part or all of the exon 5 portion of Ikzf1 gene.
[7] The composition for preventing and/or treating tumors according to [6], wherein the substance that deletes part or all of the exon 5 portion of Ikzf1 gene is selected from the group consisting of exon skipping agents for exon 5 of Ikzf1 gene, and gene therapy vectors such as CRISPR/Cas.
[8] The composition for preventing and/or treating tumors according to [7], wherein the exon skipping agent is a nucleic acid.
[9] The composition for preventing and/or treating tumors according to [1], which comprises a T cell expressing Ikzf1ΔE5.
[10] A method for preventing and/or treating tumors, which comprises administering the composition for preventing and/or treating tumors according to any one of [1] to [9].

The present invention further encompasses another aspect as follows.

### <Compositions for modulating Interferon gamma>

[21] A composition for modulating interferon gamma production, which comprises an Ikzf1ΔE5 protein or an Ikzf1ΔE5 gene-related substance.
[22] The composition for modulating the interferon gamma production according to [21], wherein the composition targets a T-cell.
[23] The composition for modulating the interferon gamma production according to [22], wherein the T-cell is a CD4-positive T-cell.
[24] The composition for modulating the interferon gamma production according to [22], wherein the T-cell is a regulatory T-cell and/or a naive T-cell.
[25] The composition for modulating the interferon gamma production according to any one of [21] to [24], wherein the Ikzf1ΔE5 gene-related substance is a nucleic acid molecule encoding the Ikzf1ΔE5 protein.
[26] The composition for modulating the interferon gamma production according to any one of [21] to [24], wherein the Ikzf1ΔE5 gene-related substance is a substance that deletes part or all of the exon 5 portion of Ikzf1 gene.
[27] The composition for modulating the interferon gamma production according to [26], wherein the substance that deletes part or all of the exon 5 portion of Ikzf1 gene is selected from the group consisting of exon skipping agents for exon 5 of Ikzf1 gene, and gene therapy vectors such as CRISPR/Cas.
[28] The composition for modulating the interferon gamma production according to [27], wherein the exon skipping agent is a nucleic acid.
[29] The composition for modulating the interferon gamma production according to [21], which comprises a T cell expressing Ikzf1ΔE5.

### <Compositions for tailoring T cells>

[31] A composition for tailoring T cells, which comprises an Ikzf1ΔE5 protein or an Ikzf1ΔE5 gene-related substance.
[32] The composition for tailoring T cells according to [31], wherein the composition targets a T-cell.
[33] The composition for tailoring T cells according to [32], wherein the T-cell is a CD4-positive T-cell.
[34] The composition for tailoring T cells according to [32], wherein the T-cell is a regulatory T-cell and/or a naive T-cell.
[35] The composition for tailoring T cells according to any one of [31] to [34], wherein the Ikzf1ΔE5 gene-related substance is a nucleic acid molecule encoding the Ikzf1ΔE5 protein.
[36] The composition for tailoring T cells according to any one of [31] to [34], wherein the Ikzf1ΔE5 gene-related substance is a substance that deletes part or all of the exon 5 portion of Ikzf1 gene.
[37] The composition for tailoring T cells according to [36], wherein the substance that deletes part or all of the exon 5 portion of Ikzf1 gene is selected from the group consisting of exon skipping agents for exon 5 of Ikzf1 gene, and gene therapy vectors such as CRISPR/Cas.
[38] The composition for tailoring T cells according to [37], wherein the exon skipping agent is a nucleic acid.
[39] The composition for tailoring T cells according to [31], which comprises a T cell expressing Ikzf1ΔE5.

The present invention further encompasses another aspect as follows.
[41] A composition for preventing and/or treating tumors, which comprises a substance for inhibiting the formation of an Ikzf1 complex containing Ikzf1 protein, and Foxp3 protein, CHD4 protein and/or HDAC1 protein, and/or a substance for promoting the formation of the Ikzf1 complex.
[42] A composition for modulating interferon gamma production, which comprises a substance for inhibiting the formation of an Ikzf1 complex containing Ikzf1 protein, and Foxp3 protein, CHD4 protein and/or HDAC1 protein, and/or a substance for promoting the formation of the Ikzf1 complex.
[43] A composition for tailoring T cells, which comprises a substance for inhibiting the formation of an Ikzf1 complex containing Ikzf1 protein, and Foxp3 protein, CHD4 protein and/or HDAC1 protein, and/or a substance for promoting the formation of the Ikzf1 complex.
[44] The composition for tailoring T cells according to [43], wherein the T cells are CD4-positive T cells.
[45] The composition for tailoring T cells according to [43], wherein the T cells are regulatory T cells and/or naive T cells.
[46] The composition according to any one of [41] to [45], wherein the Ikzf1 complex comprises Ikzf1 protein and Foxp3 protein.
[47] The composition according to any one of [41] to [46], wherein the substance for inhibiting the formation of the Ikzf1 complex comprises part or all of the exon 5 fragment of Ikzf1 protein.
[48] The composition according to any one of [41] to [47], wherein the substance for inhibiting the formation of the Ikzf1 complex comprises a nucleic acid encoding part or all of the exon 5 fragment of Ikzf1 protein.
[49] The composition according to any one of [41] to [48], wherein the substance for inhibiting the formation of the Ikzf1 complex binds to a region containing part or all of the exon 5 fragment of Ikzf1 protein.
[50] The composition according to any one of [41] to [46], wherein the substance for inhibiting the formation of the Ikzf1 complex comprises part or all of the FHD in Foxp3 protein.
[51] The composition according to any one of [41] to [46], wherein the substance for inhibiting the formation of the Ikzf1 complex comprises a nucleic acid encoding part or all of the FHD in Foxp3 protein.
[52] The composition according to any one of [41] to [46], wherein the substance for inhibiting the formation of the Ikzf1 complex binds to the region containing part or all of the FHD in Foxp3 protein.
[53] The composition according to any one of [41] to [46], wherein the substance for inhibiting the formation of the Ikzf1 complex comprises a Foxp3 protein that lacks part or all of the FHD.
[54] The composition according to any one of [41] to [46], wherein the substance for inhibiting the formation of the Ikzf1 complex comprises a nucleic acid encoding a Foxp3 protein that lacks part or all of the FHD.
[55] A method for preventing and/or treating tumors, which comprises administering the composition for preventing and/or treating tumors according to any one of [41] and [47] to [54].

### <Methods for screening substances for modulating the formation of Ikzf1 complexes>

[61] A method for screening a substance for modulating the formation of an Ikzf1 complex containing Ikzf1 protein, and Foxp3 protein, CHD4 protein and/or HDAC1 protein, which comprises the steps of:
   (a) contacting a system comprising Ikzf1 protein or a fragment thereof, and Foxp3 protein or a fragment thereof, CHD4 protein or a fragment thereof and/or HDAC1 protein or a fragment thereof with a candidate compound of a substance for modulating the formation of an Ikzf1 complex,
   (b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
   (c) identifying the candidate compound as a substance for modulating the formation of an Ikzf1 complex when the compound inhibits or promotes any one of the associations.
[62] The method for screening a substance for modulating the formation of an Ikzf1 complex according to [61], which comprises the steps of:
   (a) contacting T cells with a candidate compound of a substance for modulating the formation of an Ikzf1 complex in vitro,
   (b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
   (c) identifying the candidate compound as a substance for modulating the formation of an Ikzf1 complex when the compound inhibits or promotes any one of the associations.
[63] The method for screening a substance for modulating the formation of an Ikzf1 complex according to [61], which comprises the steps of:
   (a) contacting cultured cells or T cells with forcedly expressed Ikzf1 protein or a fragment thereof, and Foxp3 protein or a fragment thereof, CHD4 protein or a fragment thereof and/or HDAC1 protein or a fragment thereof, with a candidate compound of a substance for modulating the formation of an Ikzf1 complex in vitro,
   (b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
   (c) identifying the candidate compound as a substance for modulating the formation of an Ikzf1 complex when the compound inhibits or promotes any one of the associations.
[64] The method for screening a substance for modulating the formation of an Ikzf1 complex according to [61], which comprises the steps of:
   (a) contacting a purified Ikzf1 protein or a fragment thereof, and a purified Foxp3 protein or a fragment thereof, a purified CHD4 protein or a fragment thereof and/or a purified HDAC1 protein or a fragment thereof, with a candidate compound of a substance for modulating the formation of an Ikzf1 complex in vitro,
   (b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
   (c) identifying the candidate compound as a substance for modulating the formation of an Ikzf1 complex when the compound inhibits or promotes any one of the associations.
[65] The method according to any one of [61] to [64], wherein a substance for modulating the formation of an Ikzf1 complex is a substance for inhibiting or promoting the association of Ikzf1 protein or a fragment thereof with Foxp3 protein or a fragment thereof.
[66] The method according to any one of [61] to [65], wherein a substance for modulating the formation of an Ikzf1 complex is a composition for preventing or treating tumors, a composition for modulating interferon gamma production, or a composition for tailoring T cells.

### <Compositions for preventing and/or treating tumors: Part 2>

[71] A composition for preventing and/or treating tumors, which comprises a substance for inhibiting the activity of CHD4 protein.
[72] A composition for modulating interferon gamma production, which comprises a substance for inhibiting the activity of CHD4 protein.
[73] A composition for tailoring T cells, which comprises a substance for inhibiting the activity of CHD4 protein.
[74] The composition for tailoring T cells according to [73], wherein the T cells are CD4-positive T cells.
[75] The composition for tailoring T cells according to [73], wherein the T cells are regulatory T cells and/or naïve T cells.
[76] A method for preventing and/or treating tumors, which comprises administering the composition for preventing and/or treating tumors according to [71].

### <Compositions for preventing and/or treating tumors: Part 3>

[81] A composition for preventing and/or treating tumors, which comprises a substance for inhibiting the activity of HDAC1 protein.
[82] A composition for modulating interferon gamma production, which comprises a substance for inhibiting the activity of HDAC1 protein.
[83] A composition for tailoring T cells, which comprises a substance for inhibiting the activity of HDAC1 protein.
[84] The composition for tailoring T cells according to [83], wherein the T cells are CD4-positive T cells.
[85] The composition for tailoring T cells according to [83], wherein the T cells are regulatory T cells and/or naive T cells.
[86] A method for preventing and/or treating tumors, which comprises administering the composition for preventing and/or treating tumors according to [81].

### EFFECT OF INVENTION

According to the present invention, it is possible to provide new medicaments for preventing and treating tumors.

### BRIEF DESCRIPTION OF DRAWINGS

[FIG. 1] Figure 1 (top) depicts the structure of each Flag-Ikzf1 variant. Figure 1 (bottom) is a photograph showing the results of Western blotting in which each Ikzf1 variant and Myc-Foxp3 were forcedly expressed in HEK293 cells and immunoprecipitated with anti-Flag-M2 antibody. In the figure, α-Flag represents anti-Flag-M2 antibody, and α-Myc represents anti-c-Myc antibody.
[FIG. 2] Figure 2 (left) depicts the structure of each Foxp3 variant. Figure 2 (right) is a photograph showing the results of Western blotting in which each Foxp3 variant and Myc-Ikzf1 were forcedly expressed in HEK293 cells and immunoprecipitated with anti-Flag-M2 antibody.
[FIG. 3] Figure 3 is a photograph showing the results of Western blotting in which endogenous Foxp3 or Ikzf1 was immunoprecipitated in Ikzf1-expressing Treg cells (Foxp3^{Cre}) and Ikzf1ΔES-expressing Treg cells (Foxp3^{Cre}IKE5^{f/f}) under various conditions.
[FIG. 4] Figure 4 is a photograph showing the results of Western blotting in which HDAC1 was immunoprecipitated with endogenous Foxp3 in Ikzf1-expressing Treg cells (FYC^{+/Y}) and Ikzf1ΔES-expressing Treg cells (FYC^{+/Y}IKE5^{f/f}).
[FIG. 5] Figure 5 is a photograph showing the results of Western blotting of the immunoprecipitation with anti-Foxp3 antibody in human Treg cells.
[FIG. 6] Figure 6 is a graph comparing the percentage of Treg cells that became IFN-y positive by knockout of various Ikzfs and expression of Ikzf1ΔE5. The values were calculated by Tukey' s multiple comparisons after the usual one-way ANOVA. * P<0.05, ** P<0.01, ***; P<0.001.
[FIG. 7] Figure 7 is a graph showing the effect of Ikzf family knockout on IFN-y expression in human Treg cells.
[FIG. 8] Figure 8A is a photograph showing the results of Western blotting which confirms that Pomalidomide and REF001329 are the decomposing materials of the Ikzf family protein in human Treg cells. Figure 8B is a graph showing the percentages of IFN-γ positive Treg cells gated by flow cytometry.
[FIG. 9] Figure 9 shows the flow cytometry results of the effect of protein knockdown of Ikzf1,3 with pomalidomide on differentiation into Treg cells in human naive T cells.
[FIG. 10] Figure 10 shows the anti-tumor effect of Ikzf1ΔE5 Treg cells. Figure 10A depicts the experimental procedure. Figure 10B is a graph showing that B16FO cell engraftment was suppressed in Foxp3eGFP-Cre-ERT2IkE5^{f/f} mice expressing Ikzf1ΔES specifically in the Foxp3 cells, compared to Foxp3eGFP-Cre-ERT2 mice. Figure 10C is a graph showing that MC38 cell engraftment was suppressed in Foxp3eGFP-Cre-ERT2IkE5^{f/f} mice expressing Ikzf1ΔE5, compared to Foxp3eGFP-Cre-ERT2 mice.
[FIG. 11] Figure 11 shows the results of the percentage of exTregs measured by FACS analysis in spleens and lymph nodes collected from Foxp3^{Cre/+}R26^{RFP/+} and Foxp3^{Cre/+}IkE5^{f/f}R26^{REF/+} mice (3-4 weeks old). Foxp3-expressing Treg cells are detected by FACS analysis as both RFP- and YFP-positive cell population, and exTregs wherein Foxp3 expression is lost are detected by FACS analysis as RFP-positive and YFP-negative cell population.
[FIG. 12] Figure 12 is a normalized density plot of p300 and NFAT1 binding peaks around the Enhanced Foxp3-binding Sites in CD4+YFP+ Treg cells from Foxp3Cre mice (straight line) and Foxp3CreIkE5f/f (broken line) mice. Normalized signal density is plotted within a window ± 1 kb centered on Foxp3-binding sites.

### ASPECTS FOR CARRYING OUT INVENTION

### <Compositions for preventing and/or treating tumors>

In one aspect, the present invention relates to a composition for preventing and/or treating tumors, which comprises an Ikzf1ΔE5 protein or an Ikzf1ΔE5 gene-related substance. According to the invention, tumors mean solid tumors, epithelial cell cancers such as lung cancer, breast cancer, stomach cancer, colon cancer, liver cancer and malignant melanoma, and non-epithelial cell cancers such as osteosarcoma, chondrosarcoma, rhabdomyosarcoma, and leiomyosarcoma, as well as blood cancers such as leukemia, malignant lymphoma, and multiple myeloma.

According to the present invention, Ikzf1ΔE5 gene-related substances include not only nucleic acid molecules encoding the Ikzf1ΔE5 proteins, but also substances that delete part or all of the exon 5 portion of Ikzf1 gene in target cells. In this regard, a part of the exon 5 portion of Ikzf1 gene may mean a fragment containing 95% or more, 90% or more, 85% or more, 80% or more, 75% or more, 70% or more, 65% or more, 60% or more, 55% or more, 50% or more, 45% or more, 40% or more, 35% or more, 30% or more, 25% or more, 20% or more, 15% or more, 10% or more, or 5% or more of the exon 5 portion. Preferably, a part of the exon 5 portion of Ikzf1 gene mean a portion containing zinc finger domain 2 (ZF2) of Ikzf1, a portion containing zinc finger domain 3 (ZF3) of Ikzf1, or a portion containing both ZF2 and ZF3. Examples of target cells include T cells, preferably CD4-positive T cells, more preferably regulatory T cells or naive T cells.

According to the present invention, examples of the Ikzf1ΔE5 gene-related substance which is a substance that deletes part or all of the exon 5 portion of Ikzf1 gene include exon skipping agents for exon 5 of the Ikzf1 gene and gene therapy vectors such as CRISPR/Cas. An exon skipping agent is a substance that acts in the nucleus of a cell not to recognize (skip) the specific exon adjacent to the place where a mutation occurs in the specific mRNA precursor so as to connect the adjacent exons together, thereby creating a desired deletion form. According to the present invention, the exon skipping agent is preferably a nucleic acid, and more preferably an antisense nucleic acid.

According to the present invention, gene therapy vectors such as CRISPR/Cas are not limited to any particular ones as far as they can delete part or all of the exon 5 portion of Ikzf1 gene by genome editing, and the technology described in WO2018/179578 for example can be applied to those vectors.

In another embodiment, the present invention relates to a composition for preventing and/or treating tumors of the present invention, which comprises a T cell expressing Ikzf1ΔE5. The embodiment can be applied for example to adoptive T-cell therapy.

In another aspect, the present invention relates to a composition for preventing and/or treating tumors of the present invention, which comprises a substance for inhibiting the formation of an Ikzf1 complex containing Ikzf1 protein, and Foxp3 protein, CHD4 protein and/or HDAC1 protein, or a substance for inhibiting the activity of CHD4 protein and/or HDAC1 protein. The substances for inhibiting the formation can be screened by any well-known method. Preferably, the substances for inhibiting the formation inhibit an association between Ikzf1 protein and Foxp3 protein. As the substances for inhibiting the formation, proteins containing part or all of the exon 5 fragment of Ikzf1 protein, proteins containing part or all of the FHD of Foxp3 protein, Foxp3 proteins deficient in part or all of the FHD, or nucleic acids encoding them may also be used. In this regard, the FHD in Foxp3 means a DNA binding region of Foxp3 (Forkhead domain). According to the present invention, the FDH means preferably a C terminal region after residue 279 of Foxp3 protein represented by SEQ ID NO: 4. A part of the exon 5 fragment of Ikzf1 protein may mean a fragment containing 95% or more, 90% or more, 85% or more, 80% or more, 75% or more, 70% or more, 65% or more, 60% or more, 55% or more, 50% or more, 45% or more, 40% or more, 35% or more, 30% or more, 25% or more, 20% or more, 15% or more, 10% or more, or 5% or more of the total length of the fragment, and preferably mean a portion containing zinc finger domain 2 (ZF2) of Ikzf1, a portion containing zinc finger domain 3 (ZF3) of Ikzf1, or a portion containing both ZF2 and ZF3. A part of the FHD of Foxp3 protein may mean a fragment containing 95% or more, 90% or more, 85% or more, 80% or more, 75% or more, 70% or more, 65% or more, 60% or more, 55% or more, 50% or more, 45% or more, 40% or more, 35% or more, 30% or more, 25% or more, 20% or more, 15% or more, 10% or more, or 5% or more of the total length of the fragment, preferably a C terminal region after residue 279 of Foxp3 protein represented by SEQ ID NO: 4. Also, part or all of the exon 5 fragment of Ikzf1 protein herein encompasses a peptide in which the amino acid sequence of the corresponding portion is 95% or more, 90% or more, 85% or more, 80% or more, 75% or more, 70% or more, 65% or more, 60% or more, 55% or more is identical, and part or all of the FHD in Foxp3 protein encompasses a peptide in which the amino acid sequence of the corresponding portion is 95% or more, 90% or more, 85% or more, 80% or more, 75% or more, 70% or more, 65% or more, 60% or more, 55% or more, or 50% or more is identical.

Examples of the substance for inhibiting the activity of CHD4 protein and/or HDAC1 protein include, for example, a substance for inhibiting the chromatin remodeling activity, the ATPase activity, the DNA binding activity, and/or the deacetylation activity of CHD4 protein and/or HDAC1 protein. Substances for inhibiting the activity of CHD4 protein and/or HDAC1 protein may be purchased by any well-known methods, and may be purchased by measuring for example a chromatin remodeling activity by "Mononucleosome disruption assay" (Nucleic Acids Res. 2001 Jun 15; 29(12): 2517-21.), and screening on the basis of the measured activity.

In one embodiment, substances that bind to a region containing part or all of the exon 5 portion of Ikzf1 protein or substances that bind to a region containing part or all of the FHD of Foxp3 protein can also be used as a substance for inhibiting the formation of an Ikzf1 complex. In this regard, examples of the substance include an antibody directed to the regions as an epitope. Antibodies that can be used as a substance for inhibiting the formation of an Ikzf1 complex may be prepared by conventional methods, for example, using peptides or proteins containing part or all of the exon 5 portion of Ikzf1 protein, or part or all of the FHD of Foxp3 protein as antigens. Such antibodies may be administered as is in forms of antibody preparations, or in forms of mRNAs or gene therapy vectors such as AAV.

It should be noted that the present invention underlies the discovery that the inhibition of the formation of an Ikzf1 complex containing Ikzf1 protein, and Foxp3 protein, CHD4 protein and/or HDAC1 protein encourages cell reprogramming in T cells to improve the production of interferon gamma. Thus, the composition for preventing and/or treating tumors according to the present invention encompasses any and all aspects and embodiments as far as they inhibit the formation of an Ikzf1 complex containing Ikzf1 protein, and Foxp3 (e.g., an FHD) protein, CHD4 protein and/or HDAC1 protein.

In connection with such embodiments, the invention relates to a composition for modulating interferon gamma production, which comprises an Ikzf1ΔE5 protein, an Ikzf1ΔE5 gene-related substance, a substance for inhibiting the formation of an Ikzf1 complex as described above, a substance for promoting the formation of an Ikzf1 complex as described above (hereinafter, a substance for inhibiting the formation of an Ikzf1 complex, and a substance for promoting the formation of an Ikzf1 complex may be referred to as "a substance for modulating the formation of an Ikzf1 complex"), or a substance for inhibiting the activity of CHD4 protein and/or HDAC1 protein. According to the present invention, the modulation of interferon gamma production encompasses increased productions and decreased productions. In this regard, increased productions may mean an increase in interferon gamma production of 5% or more, 10% or more, 15% or more, 20% or more, 25% or more, 30% or more, 35% or more, 40% or more, 45% or more, or 50% or more after the treatment with a composition for modulating interferon gamma production, and decreased productions may mean a decrease in interferon gamma production of 5% or more, 10% or more, 15% or more, 20% or more, 25% or more, 30% or more, 35% or more, 40% or more, 45% or more, or 50% or more after the treatment with a composition for modulating interferon gamma production.

Interferon gamma is known to exhibit antiviral, immunomodulatory, and antitumor effects. Examples of interferon gamma-related diseases include tumors, as well as autoimmune diseases, inflammations, colitis, idiopathic pulmonary fibrosis (IPF), and the like. The present invention can be used to prevent and/or treat these interferon gamma-related diseases. The tumors here can be exemplified by those listed above. Examples of autoimmune diseases include rheumatoid arthritis, systemic lupus erythematosus, ulcerative colitis, lupus nephritis, Crohn's disease, and the like.

As described above, examples of the cells targeted according to the present invention are T cells, preferably CD4-positive T cells, and more preferably regulatory T cells or naive T cells. In this embodiment, the present invention relates to a composition for tailoring T cells, which comprises an Ikzf1ΔE5 protein, an Ikzf1ΔE5 gene-related substance, a substance for inhibiting the formation of an Ikzf1 complex as described above, or a substance for inhibiting the activity of CHD4 protein and/or HDAC1 protein. According to the present invention, a composition for tailoring T cells means an agent that changes T cells, preferably CD4-positive T cells, more preferably regulatory T cells or naive T cells, even more preferably regulatory T cells, into T cells that produce interferon gamma; an agent that suppresses the changes of T cells, preferably CD4-positive T cells, more preferably regulatory T cells or naive T cells, even more preferably regulatory T cells, into T cells that produce interferon gamma; an agent that changes T cells into T cells that decrease in Foxp3 expression (exTregs); an agent that decreases regulatory T cells; or an agent that adjusts the ration of regulatory T cells to exTregs.

As used herein, "treatment", "treating" and "treat" mean a method or process aimed at (1) delaying the onset of tumors and/or other interferon gamma-related diseases; (2) slowing or stopping the progression, aggravation or exacerbation of symptoms of tumors and/or other interferon gamma-related diseases; (3) inducing remission of the symptoms of tumors and/or other interferon gamma-related diseases; or (4) facilitating the cure of tumors and/or other interferon gamma-related diseases. Treatment and so on may be provided as a prophylactic measure before the onset of the diseases or conditions, or treatment and so on may also be provided after the onset of the diseases.

As used herein, "prevention", "preventing", and "prevent" means a prophylactic action for the development of tumors and/or other interferon gamma-related diseases.

According to the present invention, an agent or a pharmaceutical composition usually means a medicament for treating or preventing a disease, or for test/diagnosis for a disease.

The pharmaceutical composition of the present invention can be formulated according to techniques well known to those skilled in the art. For example, it may be used parenterally in an injection form of a sterile solution or a suspension with a water or another pharmaceutically acceptable liquid. For example, it is believed to formulate the composition by appropriately combining it with a pharmacologically acceptable carrier or vehicle, specifically a sterile water or a saline, a vegetable oil, an emulsifier, a suspending agent, a surfactant, a stabilizer, a flavoring agent, an excipient, a vehicle, a preservative, a binder or the like, and mixing the same to form a unit dosage form required for generally accepted pharmaceutical practice. The amounts of an active ingredient in these formulations are set so as to obtain an appropriate volume in the specified range.

Sterile compositions for injection may be prepared according to conventional formulations using vehicles such as a distilled water for injection.

Examples of aqueous solutions for injection include, for example, a saline, an isotonic solution containing glucose and other adjuvants (such as D-sorbitol, D-mannose, D-mannitol, and sodium chloride). Appropriate solubilizing agents such as an alcohol (ethanol, and so on), a polyalcohol (propylene glycol, polyethylene glycol, and so on), and a nonionic surfactant (polysorbate 80 (TM), HCO-50, and so on) may be used together.

Examples of oily liquids include a sesame oil and a soybean oil, and they may be used together with benzyl benzoate and/or benzyl alcohol as a solubilizing agent. They may also be blended with a buffer (such as a phosphate buffer and a sodium acetate buffer), a soothing agent (such as procaine hydrochloride), a stabilizer (such as benzyl alcohol and phenol), or an antioxidant. The prepared injection solutions are usually filled in a suitable ampoule.

The pharmaceutical composition of the present invention is preferably administered by parenteral route. For example, the composition may be an injection dosage form, a transnasal administration dosage form, a pulmonary administration dosage form, or a transdermal administration dosage form. For example, the composition may be administered systemically or topically by intravenous injection, intramuscular injection, intraperitoneal injection, subcutaneous injection, or the like.

The administration method may be appropriately selected depending on the age and symptoms of a patient. The dose of the pharmaceutical composition comprising the polypeptide may be set, for example, in the range of 0.0001 mg to 1000 mg per kg of body weight at one time. Alternatively, for example, the dose may be set in 0.001 to 100,000 mg per patient, but the present invention is not necessarily limited to these values. The dose and the administration method vary depending on the weight, age, symptom, and the like of a patient, and those skilled in the art can set an appropriate dose and an administration method in consideration of these conditions.

### <Screening Method>

In another aspect, the present invention relates to a method for screening a substance for modulating the formation of an Ikzf1 complex containing Ikzf1 protein, and Foxp3 protein, CHD4 protein and/or HDAC1 protein, which comprises the steps of:
(a) contacting a system comprising Ikzf1 protein or a fragment thereof, and Foxp3 protein or a fragment thereof, CHD4 protein or a fragment thereof and/or HDAC1 protein or a fragment thereof with a candidate compound of a substance for modulating the formation of an Ikzf1 complex,
(b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
(c) identifying the candidate compound as a substance for modulating the formation of an Ikzf1 complex when the compound inhibits or promotes any one of the associations.

Preferably, a substance for modulating the formation of an Ikzf1 complex means a substance for inhibiting or promoting the association of Ikzf1 protein or a fragment thereof with Foxp3 protein or a fragment thereof. Also, the substance for modulating the formation of an Ikzf1 complex includes a composition for preventing or treating tumors, a composition for modulating interferon gamma production, or a composition for tailoring T cells.

In another view of the embodiment, the present invention relates to a method for screening a substance for modulating the formation of an Ikzf1 complex containing Ikzf1 protein, and Foxp3 protein, CHD4 protein and/or HDAC1 protein, which comprises the steps of:
(a) contacting T cells with a candidate compound of a substance for modulating the formation of an Ikzf1 complex in vitro,
(b) determining whether the candidate compound inhibits or promotes the association of Ikzf1 protein with Foxp3 protein, inhibits or promotes the association of Ikzf1 protein with CHD4 protein, inhibits or promotes the association of Ikzf1 protein with HDAC1 protein, inhibits or promotes the association of Foxp3 protein with CHD4 protein, and/or inhibits or promotes the association of Foxp3 protein with HDAC1 protein, and
(c) identifying the candidate compound as a substance for modulating the formation of an Ikzf1 complex when the compound inhibits or promotes any one of the associations.

In another embodiment, the present invention relates to a method for screening a substance for modulating the formation of an Ikzf1 complex containing Ikzf1 protein, and Foxp3 protein, CHD4 protein and/or HDAC1 protein, which comprises the steps of:
(a) contacting cultured cells or T cells with forcedly expressed Ikzf1 protein, and Foxp3 protein, CHD4 protein and/or HDAC1 protein, with a candidate compound of a substance for modulating the formation of an Ikzf1 complex in vitro,
(b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
(c) identifying the candidate compound as a substance for modulating the formation of an Ikzf1 complex when the compound inhibits or promotes any one of the associations.

In the embodiment, a fragment of Ikzf1 protein means a polypeptide comprising a part of Ikzf1 protein (SEQ ID NO: 3), preferably a polypeptide comprising a part or all of an exon 5 fragment of Ikzf1 protein. A fragment of Foxp3 protein means a polypeptide comprising a part of Foxp3 protein (SEQ ID NO: 4), preferably a polypeptide comprising a C terminal region after residue 279 of Foxp3 protein represented by SEQ ID NO: 4.

In the embodiment, an association of proteins may be estimated by any well-known methods, and examples of such methods include NanoLuc (Trade Mark) Binary Technology (NanoBiT), bioluminescence resonance energy transfer (BRET), chemically amplified luminescence proximity homogeneous assay (Alpha), TR-FRET, and the like.

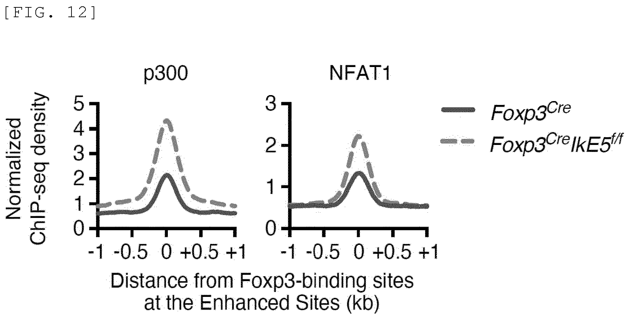

The Ikzf1 complex according to the embodiment not only promotes the expression of its target genes, but also suppresses the transcription of its target genes by competing with co-activators such as p300, a histone acetyltransferase (HAT), and NFAT1 (nuclear factor of activated T cells 1), which is responsible for transcriptional regulation of the cytokine IL-2 in activated T cells, said suppression being released by inhibition of the formation of the Ikzf1 complex (Fig. 12). Therefore, the target genes or corresponding promoters of the Ikzf1 complex and the target genes or corresponding promoters of p300 and NFAT1 can be used as reporters for screening substances for modulating the formation of an Ikzf1 complex.

In this regard, examples of target genes of the Ikzf1 complex include ones described in J Biol Chem. 2001 Jul 20;276(29):27647-56, examples of target genes of p300 include ones described in J Biol Chem. 2001 Jul 20;276(29):27647-56, and examples of target genes of NFAT1 include ones described in J Biol Chem. 2014 Sep 26;289(39):26752-26761. The corresponding promoters are also used in the above screening in the form of a vector coupled with a reporter gene. In this regard, examples of the reporter genes include genes encoding known reporter proteins such as β-galactosidase, β-gluconidase, luciferase, Green Fluorescent Protein (GFP), tdTomato, cell surface proteins and the like.

In another view of the embodiment, the present invention relates to a method for screening a substance for modulating the formation of an Ikzf1 complex containing Ikzf1 protein, and Foxp3 protein, CHD4 protein and/or HDAC1 protein, which comprises the steps of:
(a) contacting a purified Ikzf1 protein or a fragment thereof, and a purified Foxp3 protein or a fragment thereof, a purified CHD4 protein or a fragment thereof and/or a purified HDAC1 protein or a fragment thereof, with a candidate compound of a substance for modulating the formation of an Ikzf1 complex in vitro,
(b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
(c) identifying the candidate compound as a substance for modulating the formation of an Ikzf1 complex when the compound inhibits or promotes any one of the associations.

In this embodiment, the degree of the association between the purified proteins can be evaluated using any well-known methods, such as immunoprecipitation, pull-down assay, Western blotting, NMR, Surface Plasmon Resonance (SPR), gel shift assay, gel filtration, microscale thermophoresis (MST) and the like. In general, it is preferable that the same assay system should be performed in the absence of a candidate compound, and the interactions in both the presence and absence of the candidate compound should be measured and compared to determine whether the candidate compound inhibits or promotes the association of the target protein.

In the embodiment, a fragment of Ikzf1 protein means a polypeptide comprising a part of Ikzf1 protein (SEQ ID NO: 3), preferably a polypeptide comprising a part or all of an exon 5 fragment of Ikzf1 protein. A fragment of Foxp3 protein means a polypeptide comprising a part of Foxp3 protein (SEQ ID NO: 4), preferably a polypeptide comprising a C terminal region after residue 279 of Foxp3 protein represented by SEQ ID NO: 4.

T cells used in the screening methods of the present invention may be prepared by techniques well known in the art, and for example the cells may be obtained by blood collection from patients or healthy individuals, or by biopsy, or may be purchased from immune and microbial suppliers, such as the American Type Culture Collection, Manassas, VA.

T cells as used according to the present invention may be cultured by standard cell culture techniques. For example, cells are grown in a sterile environment at 37°C in an incubator containing humidified 95% air - 5% CO₂ in a suitable vessel. Vessels can contain agitated or static media. Various cell culture media may be used, which comprise media containing incomplete biological fluids (such as fetal bovine serum) and fully complete media such as 293 SFM serum-free medium (Invitrogen Corp., Carlsbad, CA). Cell culture techniques are well known in the art, and established protocols are available for the culture of diverse cell types (See, for example, R.I. Freshney, "Culture of Animal Cells: A Manual of Basic Technique", 2nd Edition, 1987, Alan R. Liss, Inc.).

In a certain embodiment, the screening method of the present invention is performed using cells contained in multiple wells of a multi-well assay plate. Such assay plates are commercially available, for example, from Strategene Corp. (La Jolla, CA) and Corning Inc. (Acton, MA), which commercial products include 48-well, 96-well, 384-well, and 1536-well plates.

"Contacting in vitro" means, for example, adding a candidate compound adjusted to a given concentration to cells in multiple wells of a multi-well assay plate. The candidate compound is then examined for its inhibitory activity for the association between the target proteins. In this regard, the inhibitory activity for the association may be examined by any well-known method, and the activity may be examined by for example an interaction test, which determines whether or not one of the target proteins co-precipitates with the other protein that has been immunoprecipitated, or by NanoBit or TR-FRET related techniques.

The activity of CHD4 protein and/or HDAC1 protein may be measured by any well-known methods, and, for example, a chromatin remodeling activity may be measured by "Mononucleosome disruption assay" (Nucleic Acids Res. 2001 Jun 15; 29(12): 2517-21.).

### Examples

Hereinafter, the present invention will be described in more detail by way of the working examples, but it should be noted that these would not limit the scope of the present invention and merely illustrate the invention.

The antibodies used in the working examples are as follows.

**Table 1**

| Mouse | Product names | Suppliers |
|---|---|---|
| IP | Monoclonal ANTI-FLAG M2 antibody produced in mouse | Sigma-Aldrich |
| | Myc-Tag (71D10) Rabbit | Cell Signaling Technology |
| | Normal Mouse IgG | Sigma-Aldrich |
| | FOXP3 Monoclonal Antibody (150D/E4) | eBioscience |
| IB | Anti-CHD4 antibody[EPR22953-38]-ChIP Grade | Abcam |
| ICS | V450 Rat Anti-Mouse IFN-g | BD Horizon |
| Cell culture | Purified NA/LE Hamster Anti-Mouse CD3e | BD Pharmingen |
| | Purified NA/LE Hamster Anti-Mouse CD28 | BD Pharmingen |
| | | |

| Human | Product names | Suppliers |
|---|---|---|
| IP | FOXP3 Monoclonal Antibody (PCH101) | eBioscience |
| | Rat IgG2a kappa Isotype Control (eBR2a) | eBioscience |
| IB | Ikaros (D6N9Y) Rabbit mAb | CST |
| | Helios (D8W4X) XP^{®} Rabbit mAb | CST |
| | Aiolos (D1C1E) Rabbit mAb | CST |
| | Rabbit anti-ZNFN1A4 pAb | Novus |
| | beta Actin Monoclonal Antibody (AC-15) | Invitrogen |
| ICS | PE Mouse Anti-Human CD25 | BD Biosciences |
| | PerCP/Cyanine5.5 anti-human CD4 Antibody | Biolegend |
| | Brilliant Violet 605^{™} anti-human CD45RA Antibody | Biolegend |
| | Brilliant Violet 421^{™} anti-human IFN-γ Antibody | Biolegend |
| | APC anti-mouse/human Helios Antibody | Biolegend |
| | PE/Cyanine7 anti-human Ikaros Antibody | Biolegend |
| | KIRAVIA Blue 520^{™} anti-human FOXP3 Antibody | Biolegend |

### Example 1

### Analysis for Ikzf1-Foxp3 association

Foxp3 and Ikzf1 interact as described in Nat Immunol. 2012 Oct; 13(10): 1010-9. Therefore, each of the regions required for the Foxp3 and Ikzf1 interactions was identified as follows.

### Example 1-1: Identification of the respective regions of Foxp3 and Ikzf1 in Foxp3-Ikzf1 complex formation

First, the region of Ikzf1 for association with Foxp3 was identified.

Specifically, expression vectors encoding Myc-Foxp3 and Flag-Ikzf1 variants: Flag-Ikzf1 (Ik1), Flag-IkΔN, Flag-IkΔC, Flag-IkΔE4, Flag-IkΔE6, and Flag-IkΔE5 were transfected into HEK293T cells (human fetal kidney cells) with FuGENE HD Transfection Reagent (Promega). Forty-eight hours after the gene transfection, the cells were collected in immunoprecipitation cell lysis buffer (IP lysis buffer). Immunoprecipitation was performed using anti-Flag-M2 antibody overnight at 4°C, and the cell lysates (Input) wherein the protein levels were equalized and the immunoprecipitated samples (IP) were electrophoresed, and analyzed by Western blotting (Western Blot) using anti-Flag-M2 or anti-c-Myc antibody.

The amino acid sequence of the DNA binding protein Ikaros transcript variant 7 [Mus musculus] used to prepare the Flag-Ikzf1 variants is as follows:

MDVDEGQDMSQVSGKESPPVSDTPDEGDEPMPVPEDLSTTSGAQQNSKSDRGMVAYGAD

GFRDFHAIIPKSFSPSNVKVETQSDEENGRACEMNGEECAEDLRMLDASGEKMNGSHRD

QGSSALSGVGGIRLPNGKLKCDICGIVCIGPNVLMVHKRSHTGERPFQCNQCGASFTQK

GNLLRHIKLHSGEKPFKCHLCNYACRRRDALTGHLRTHSVGKPHKCGYCGRSYKQRSSL

EEHKERCHNYLESMGLPGMYPVIKEETNHNEMAEDLCKIGAERSLVLDRLASNVAKRKS

SMPQKFLGDKCLSDMPYDSANYEKEDMMTSHVMDQAINNAINYLGAESLRPLVQTPPGS

SEVVPVISSMYQLHKPPSDGPPRSNHSAQDAVDNLLLLSKAKSVSSEREASPSNSCQDS

TDTESNAEEQRSGLIYLTNHINPHARNGLALKEEQRAYEVLRAASENSQDAFRVVSTSG

EQLKVYKCEHCRVLFLDHVMYTIHMGCHGFRDPFECNMCGYHSQDRYEFSSHITRGEHR

YHLS (SEQ ID NO: 1)

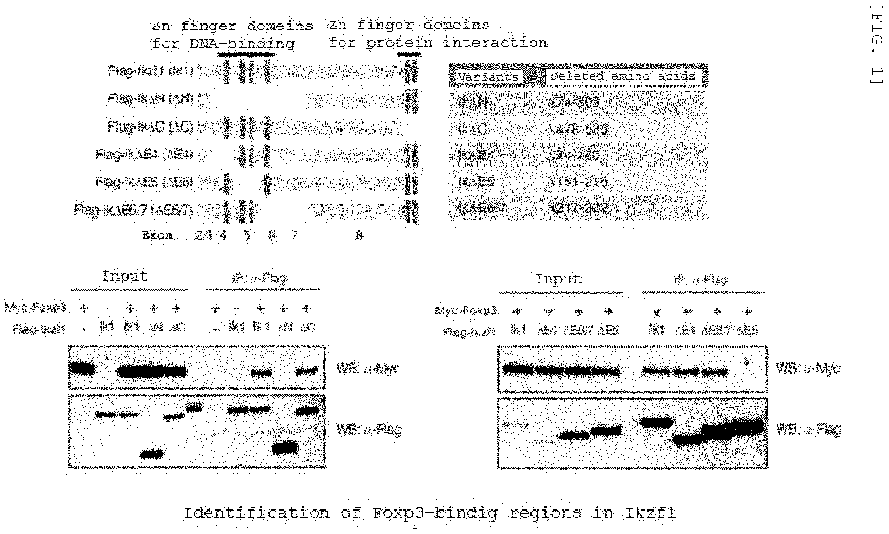

The results are shown in Figure 1. Figure 1, illustrating whether Foxp3 is co-precipitated when each Ikzf1 variant was immunoprecipitated with anti-Flag-M2 antibody, showed that Foxp3 did not co-precipitate with the ΔN and ΔE5 variants. This revealed that the region encoded by exon 5 of Ikzf1 interacts with Foxp3.

Next, the region of Foxp3 for association with Ikzf1 was identified.

Specifically, expression vectors encoding Myc-Ikzf1 and the Flag-Foxp3 variants: Flag-Foxp3, Flag-ΔNFoxp3 and Flag-ΔCFoxp3 were used to conduct the studies in a similar manner to the above.

The amino acid sequence of the forkhead box P3 [Mus musculus] used to prepare the Flag-Foxp3 variants is as follows:

MPNPRPAKPMAPSLALGPSPGVLPSWKTAPKGSELLGTRGSGGPFQGRDLRSGAHTSSS
LNPLPPSQLQLPTVPLVMVAPSGARLGPSPHLQALLQDRPHFMHQLSTVDAHAQTPVLQ
VRPLDNPAMISLPPPSAATGVFSLKARPGLPPGINVASLEWVSREPALLCTFPRSGTPR
KDSNLLAAPQGSYPLLANGVCKWPGCEKVFEEPEEFLKHCQADHLLDEKGKAQCLLQRE
VVQSLEQQLELEKEKLGAMQAHLAGKMALAKAPSVASMDKSSCCIVATSTQGSVLPAWS
APREAPDGGLFAVRRHLWGSHGNSSFPEFFHNMDYFKYHNMRPPFTYATLIRWAILEAP
ERQRTLNEIYHWFTRMFAYFRNHPATWKNAIRHNLSLHKCFVRVESEKGAVWTVDEFEF
RKKRSQRPNKCSNPCP (SEQ ID NO: 2)

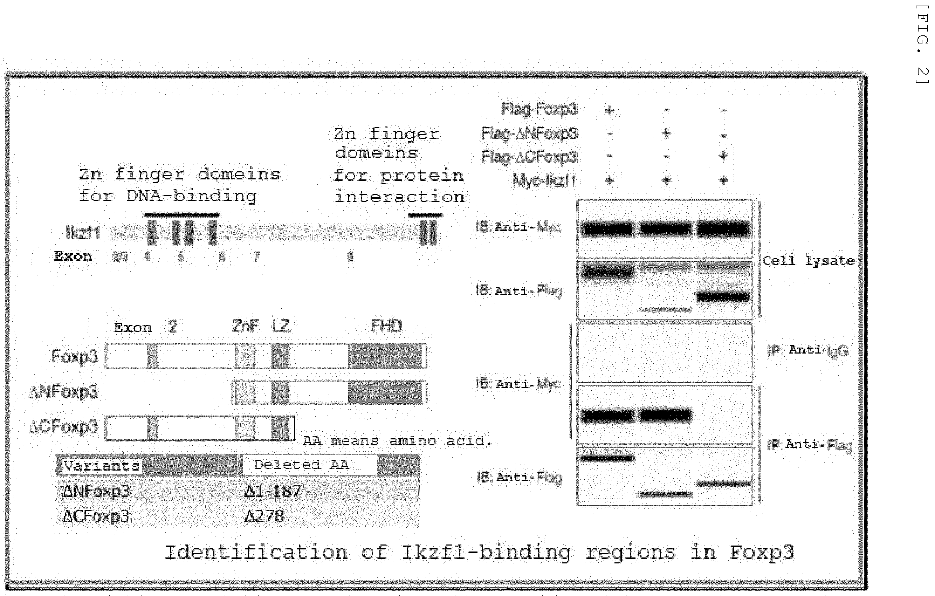

The results are shown in Figure 2. Figure 2, illustrating whether Ikzf1 is co-precipitated when each Foxp3 variant was immunoprecipitated with anti-Flag-M2 antibody, showed that Ikzf1 did not co-precipitate with ΔCFoxp3 (rightmost lane). This revealed that the FHD of Foxp3 interacts with Ikzf1.

### Example 1-2: Analysis for Ikzf1 complex using Treg cells derived from Foxp3^{Cre}IkE5^{f/f} mice

In Foxp3^{Cre}IkE5^{f/f} mice, which are derived by crossing IkE5^{f/f} mice (joshi et al., Nat. Immunol. 2014) with Foxp3^{Cre} transgenic mice (rudensky et al., Immunity 2008), exon 5 of Ikzf1 gene (IkE5) is removed by homologous recombination in Foxp3-positive Treg cells, and Ikzf1ΔE5 expression is induced. YFP⁺Treg cells (1×10⁵) prepared from the Foxp3^{Cre}IkE5^{f/f} mice were cultured in the presence of TCR stimulation and IL-2 (1500 U/ml) for 7 days, and then lysed in immunoprecipitation cell lysis buffer. Immunoprecipitation was performed using anti-IgG or anti-Foxp3 antibody (5 µg/sample) overnight at 4°C. The cell lysates (Input) wherein the protein levels were equalized and the immunoprecipitated samples (IP) were electrophoresed, and analyzed by Simple Western assay using anti-CHD4 antibody as a primary antibody. As a control, Treg cells obtained in the same way from Foxp3^{Cre} transgenic mice were used.

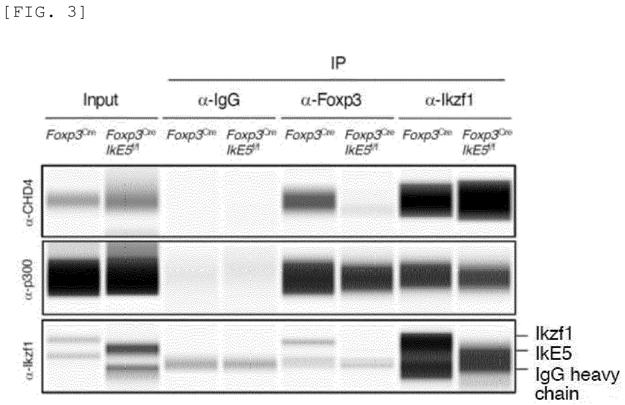

The results are shown in Figure 3. When endogenous Foxp3 was immunoprecipitated (lane: α-Foxp3), CHD4 co-precipitated in Ikzf1-expressing Treg cells (Foxp3^{Cre}) (left), whereas CHD4 did not co-precipitate in Ikzf1ΔE5-expressing Treg cells (Foxp3^{Cre}IKE5^{f/f}) (right). On the other hand, when Ikzf1 was immunoprecipitated (lane: α-Ikzf1), CHD4 co-precipitated in both Ikzf1-expressing Treg cells and Ikzf1ΔE5-expressing Treg cells. This revealed that CHD4 behaves with Ikzf1 in the Ikzf1 complex, but not with Foxp3.

### Example 1-3: Analysis for Ikzf1 complex using Treg cells derived from Foxp3^{Cre}IkE5^{f/f} mice

CD4+FYP+ Treg cells (1 × 10⁵) purified from the spleen and lymph nodes of the Foxp3^{Cre}IkE5^{f/f} mice were incubated with Dynabeads mouse CD3/CD28 T cell stimulator (25 µl/ml) (Thermo Fisher Scientific) and IL-2 (1500 U/ml) for 7 days. On day 4 during the incubation, the culture medium (containing also the cultured cells) was divided into two portions and the culture medium containing IL-2 (1500 U/ml) was added thereto at 100 µl/well. After incubation, the activated Treg cells were stimulated with Cell Stimulation Cocktail (eBioscience) for 1 hour at 37°C and lysed with immunoprecipitation cell lysis buffer (Thermo Fisher Scientific). After cell lysis, DynaBeads IgG magnetic beads (50 µl/sample) (Thermo Fisher Scientific) and anti-IgG (sigma-Aldrich) 5 µg or anti-Foxp3 antibody (eBioscience) 5 µg were added thereto and the mixture was immunoprecipitated at 4°C overnight. After immunoprecipitation, the DynaBeads were washed 5 times with wash buffer (1 ml/sample), and after washing, the DynaBeads were suspended in sample buffer (25 µl/sample) to collect proteins. Equal amounts of the proteins from the whole cell lysates (Input) or the immunoprecipitates (IP) were analyzed by Simple Western assay. As a control, Treg cells similarly obtained from Foxp3^{Cre} mice were used to compare the changes in interaction with Foxp3 by induced expression of Ikzf1ΔE5.

Simple Western assay was performed using Jess Simple Western System (ProteinSimple) according to the manufacturer's standard method.

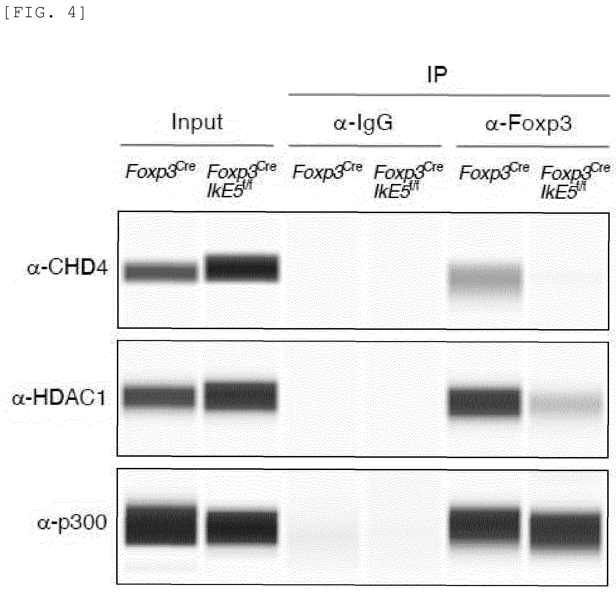

The results are shown in Figure 4. When endogenous Foxp3 was immunoprecipitated (lane of α-Foxp3), CHD4 and HDAC1 were co-precipitated in Ikzf1-expressing Treg cells (Foxp3^{Cre}) (left), whereas CHD4 was not co-precipitated and HDAC1 was decreased in Ikzf1ΔE5-expressing Treg cells (Foxp3^{Cre}IkE5^{f/f}) (right). This revealed that HDAC1 behaves with Ikzf1, but not with Foxp3 in the Ikzf1 complex, similarly to CHD4.

### Example 1-4: Interaction between Foxp3 and Ikzf1 in human Treg cells

Human Treg cells (STEMCELL TECHNOLOGIES) were centrifuged (600 x g, 5 minutes) after cell counting. After removal of the supernatant, the pellet of the cells was suspended in PBS and centrifuged (600 x g, 5 minutes). After centrifugation, the supernatant was removed, and 0.6 mL of RIPA buffer (Nacalai Tesque) was added to the pellet of the human Treg cells, followed by allowing the same to stand (4°C, 10 minutes). The samples were then centrifuged (15,000 rpm, 10 minutes), and each 300 µL of the supernatant was divided into 1.5-mL tubes, to which tube, 6 µg of anti-rat IgG2a antibody (eBioscience) or anti-Foxp3 antibody (eBioscience) and 20 µL of protein G Dynabeads (proteinG Dynabeads; Invitrogen) were added, followed by rotating the same with the rotator (4°C, 180 minutes). After rotation, the samples were placed on a magnet to remove the supernatant without absorbing the Dynabeads trapped by the magnet, and the process of addition of new RIPA buffer was repeated three times. Then, 25 µL of sample buffer (Thermo) was added to the samples and the mixtures were heated (95°C, 5 minutes). Samples were electrophoresed by SDS-PAGE, and Foxp3 and Ikzf1 were detected by Western blotting.

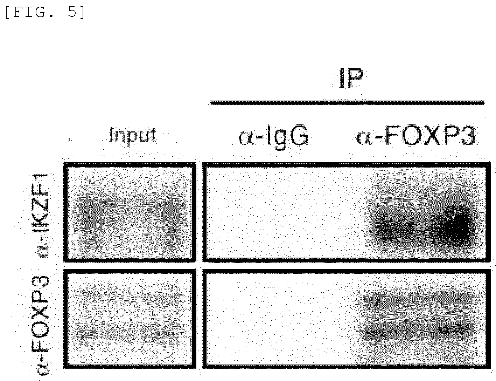

The results are shown in Figure 5. Figure 5 shows that Ikzf1 was co-precipitated when Foxp3 was immunoprecipitated with anti-Foxp3 antibody. This means that Ikzf1 and Foxp3 interacted in human Treg cells similarly in mouse Treg cells. Furthermore, in view of the fact that human Ikzf1 and Foxp3 also have the exon 5 region and FHD regions, respectively, it should be extremely likely that these regions contribute to the interaction between Ikzf1 and Foxp3 in human.

The amino acid sequences of human Ikzf1, exon 5, and Foxp3 are as follows.
Amino acid sequence of DNA-binding protein Ikaros transcript variant X5 [Homo sapience]: 
MDADEGQDMSQVSGKESPPVSDTPDEGDEPMPIPEDLSTTSGGQQSSKSDRVVVTYGAD

DFRDFHAIIPKSFSPSNVKVETQSDEENGRACEMNGEECAEDLRMLDASGEKMNGSHRD

QGSSALSGVGGIRLPNGKLKCDICGIICIGPNVLMVHKRSHTGERPFQCNQCGASFTQK

GNLLRHIKLHSGEKPFKCHLCNYACRRRDALTGHLRTHSVGKPHKCGYCGRSYKQRSSL

EEHKERCHNYLESMGLPGTLYPVIKEETNHSEMAEDLCKIGSERSLVLDRLASNVAKRK
 
SSMPQKFLGDKGLSDTPYDSSASYEKENEMMKSHVMDQAINNAINYLGAESLRPLVQTP

PGGSEVVPVISPMYQLHKPLAEGTPRSNHSAQDSAVENLLLLSKAKLVPSEREASPSNS

CQDSTDTESNNEEQRSGLIYLTNHIAPHARNGLSLKEEHRAYDLLRAASENSQDALRVV

STSGEQMKVYKCEHCRVLFLDHVMYTIHMGCHGFRDPFECNMCGYHSQDRYEFSSHITR

GEHRFHMS (SEQ ID NO: 3);
Amino acid sequence of Human Ikzf1 Exon5:
   GERPFQCNQCGASFTQKGNLLRHIKLHSGEKPFKCHLCNYACRRRDALTGHLRTHS (SEQ ID NO: 10);
Amino acid sequence of Human Ikzf1 ZF2:
   QCNQCGASFTQKGNLLRHIKLHS (SEQ ID NO: 17)
Amino acid sequence of Human Ikzf1 ZF3:
   KCHLCNYACRRRDALTGHLRTHS (SEQ ID NO: 18); and
Amino acid sequence of Foxp3 [Homo sapience]:















 (SEQ ID NO: 4).

### Example 2

### Changes to IFN-γ-positive Treg cells by effect on Ikzf in mice

### Example 2-1: Analysis for Treg cells of Ikzf1ΔE5

Cell suspensions were prepared from the spleen and lymph nodes of the Foxp3^{Cre}IkE5^{f/f} mice. The cells were suspended in FACS buffer (PBS, 2% fetal bovine serum) containing an antibody against CD4 (BD Pharmingen), and the suspension was stained on ice for 15 minutes. CD4⁺YFP⁺ Treg cells were then sorted by FACSAria II cytometer (BD). To prepare samples for staining intracellular proteins, Treg cells were stimulated in the presence of PMA/ionomycin and Brefeldin A for 4 hours, the cells were washed, and fixed in Foxp3-staining buffer (eBioscience) (45 minutes on ice). The fixed cells were washed with Perm buffer and stained with anti-IFN-γ antibody. Then, data were acquired on a FACSCanto flow cytometer (BD) and were analyzed on a FlowJo (TreeStar). As a control, Treg cells similarly obtained from Foxp3^{Cre} transgenic mice, which expressed Ikzf1, were used to compare the change to IFN-γ positive Treg cells by induction of Ikzf1ΔE5 expression.

### Example 2-2: Change of Mouse Treg Cells into IFN-γ-Positive Treg Cells by Ikzf Family Knockout

The guide RNA (gRNA) for the target gene used for knockout was designed using the IDT guide RNA design tool (lower panel). YFP⁺Treg cells (1 x 10⁵) were prepared from Foxp3^{Cre} mice and cultured in the presence of TCR (Dynabeads) and IL-2 (1500 U/ml) for 7 days. The cultured Treg cells (2 × 10⁶) were suspended in P4 primary cell solution (P4 Primary cell 4D-nucleofector X kit S) and transfected with Cas9 protein and gRNA with Amaxa 4D (DG137 program). After the transfection, the cells were washed and cultured in the presence of TCR (Dynabeads) and IL-2 (1500 U/ml) for 3 days. Then, the cell surface and the intracellular molecules were stained. As a control, gRNA (INTEGRATED DNA TECHNOLOGIES) was used. According to this method, the changes to IFN-γ positive Treg cells in Treg cells without knockout of the Ikzf family, Treg cells with knockout of only Ikzf1, and Treg cells with knockout of Ikzf1 and 3 were compared.

Sequence of gRNA used for knockout:
Ikzf1: AGAGCGATGCCACAACTACTTGG (SEQ ID NO: 5), and
CAGAACTCCAAGAGTGATCGAGG (SEQ ID NO: 6):
Ikzf3: AGATGAACTGCGACGTGTGCGGG (SEQ ID NO: 7) and
ATTATGAAGCCGGAGCCCATGGG (SEQ ID NO: 8), and
CGACTATGAAAGCATTAAGCTGG (SEQ ID NO: 9);
Ikzf2: CGAAGGGGAACACGCCAATATGG (SEQ ID NO: 11) and
GGGTAAAAGAAGCTCCGCACTGG (SEQ ID NO: 12) and
TCAGTTTACCATTCGGAAGCCGG (SEQ ID NO: 13); and
Ikzf4: AAGCTCAAGTGCGACGTCTGCGG (SEQ ID NO: 14) and
CTTGTAGGGTTTGCCCACGGTGG (SEQ ID NO: 15) and
TTATCCAGGAGCCGTTCATCCGG (SEQ ID NO: 16).

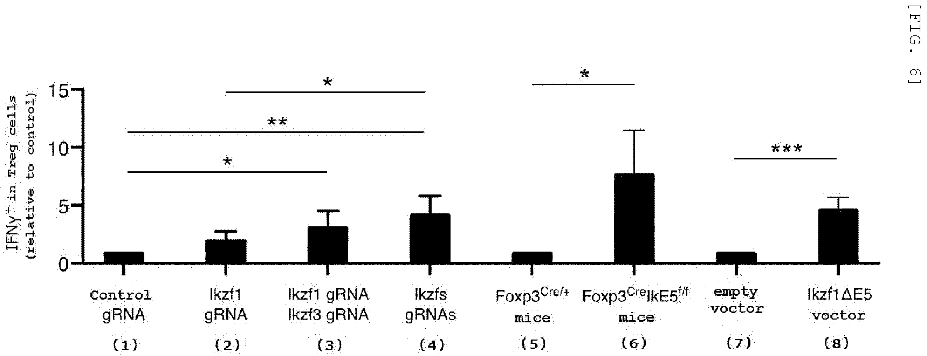

The results obtained in Examples 2-1 and 2-2 are summarized in Figure 6 (1)-(6). Figure 6 shows that the effects of Treg cells → IFN-γ positive Treg cells (IFN-γ production) caused by the action on Ikzf are in the following order, and that the change ratio to IFN-γ positive Treg cells in IkzfΔE5 expressing Treg cells was significantly higher: Ikzf1ΔE5 expression > Ikzfs (Ikzf1-4) KO > Ikzf1,3 KO > Ikzf1 KO.

As such, it has found that the expression of Ikzf1ΔE5 provides a higher change ratio to IFN-γ positive Treg cells and facilitates a higher IFN-γ production in Treg cells than knockout of Ikzf1, rather than knockout of Ikzf1,3, or Ikzf1-4.

### Example 2-3: Change of mouse Treg cells to IFN-γ-positive Treg cells by overexpression of Ikzf1ΔE5

To create retroviruses, HEK293T cells (2 × 10⁵ cells/well in 2 ml medium) were incubated for 24 hours. Serum-free medium (Opti-MEM: Gibco) was dispensed into 1.5 ml tubes at 200 µl/well, to which retroviral vectors incorporated with Ikzf1ΔE5 (1 µg) were added along with pCL-Eco (1 µg), a retroviral packaging vector. Then, 6 µl/tube volume of HD (Promega) was added thereto, and the tubes were vortexed gently, followed by allowing them to stand at room temperature for 20 minutes. After 20 minutes, serum-free medium containing the vector was added dropwise to the incubated HEK293T cells and the cells were incubated for another 2 days. After 2 days, the culture supernatant was collected and filtered with a 0.45-µm filter, which filtrates were used as virus comprising the target gene in the following experiments.

YFP⁺Treg cells were prepared from the Foxp3^{Cre} mice and incubated in 96-well plates coated with anti-CD3 antibody (1 µg/ml) and anti-CD28 antibody (1 µg/ml) in the presence of IL-2 (100 U/ml) for 24-28 hours (1 × 10⁵/well/100 µl). After the incubation, polybrene (1.5 µl/ml) was added to the already created retrovirus and the retrovirus was slowly added to the wells during incubation at a rate of 200 µl/well. After adding the retrovirus, viral infection was performed by centrifugation at 32°C, 2500 rpm for 90 minutes at a minimum accelerator and a minimum brake. After the centrifugation, the cells were placed in an incubator at 37°C for 30 minutes, then the supernatant was slowly removed (240 µl), and 200 µl of culture medium was slowly added thereto. After adding the medium, the cells were centrifuged at 32°C and 500g for 5 minutes, the supernatant was slowly removed (200 µl), and 200 µl of culture medium was slowly added thereto, followed by incubating the cells at 37°C for 4 days. On day 2 during the incubation, half volume (120 µl/well) of the culture medium (containing the cultured cells) was removed, and the same volume of culture medium was added thereto.

After the incubation, cell surface and intracellular molecules were stained. As a control, an empty retroviral vector was used, which were not incorporated with any genes. This procedure was used to compare the change of Treg cells overexpressing Ikzf1ΔE5 to IFN-γ positive Treg cells.

The results are shown in Figure 6 (7) and (8). The results here indicate that overexpression of the foreign Ikzf1ΔE5 also promotes the change to IFN-γ positive Treg cells similarly to the Ikzf1ΔE5 expression by homologous recombination, and that the change ratio is higher than that of Ikzf1 KO. The results here indicate that the expression of Ikzf1ΔE5 provides a higher change ratio to IFN-γ positive Treg cells and facilitates a higher IFN-γ production in Treg cells than knockout of Ikzf1, similarly to the results in Example 2-1 and 2-2. Furthermore, in view of the fact that Ikzf1ΔE5 is an Ikzf1 variant in which the interaction region with Foxp3 is deleted, it has been suggested that the formation of the Ikzf1 complex was inhibited by inhibiting the association between Ikzf1 and Foxp3 via the competition with Ikzf1, thereby inducing the change to IFN-γ positive Treg cells, and that the induction ability is larger than that in Ikzf1 KO.

### Example 3

### Change to IFN-γ-positive Treg cells by action on Ikzf in human

### Example 3-1: Change to IFN-γ-positive Treg cells by Ikzf family knockout in human Treg cells

EasySep Human CD4⁺CD127lowCD25⁺ Regulatory T Cell Isolation Kit (STEMCELL) was used to prepare human Treg cells. The Treg cells were cultured in the presence of Dynabeads T-activator (2.5 µl/well, Thermo Fisher Scientific) and IL-2 (100 u/ml, Miltenyi Biotec) for 9 days. The cultured Treg cells were collected. Negative control gRNA or Ikzf1, 2, 3 and 4 gRNAs was mixed with Cas9 protein (Thermo Fisher Scientific) and the mixtures were introduced into the Treg cells by Nucleofection. The next day, the Treg cells were collected and seeded into 96-well plates at 1 × 10⁴ cells/well and cultured in the presence of 100 U/ml IL-2 and 2.5 µl/well of Dynabeads T-activator. After 3 days of the incubation, Brefeldin A and Monensin, and Cell Activation Cocktail (Biolegend) were added thereto. After 5 hours of incubation, the cells were stained with anti-CD4 and anti-CD25 antibodies. After fixing the cells, IFN-γ and a nuclear protein Foxp3 were stained, and the percentage of Treg cells expressing IFN-γ was analyzed with CytoFLEX LX (Beckman Coulter).

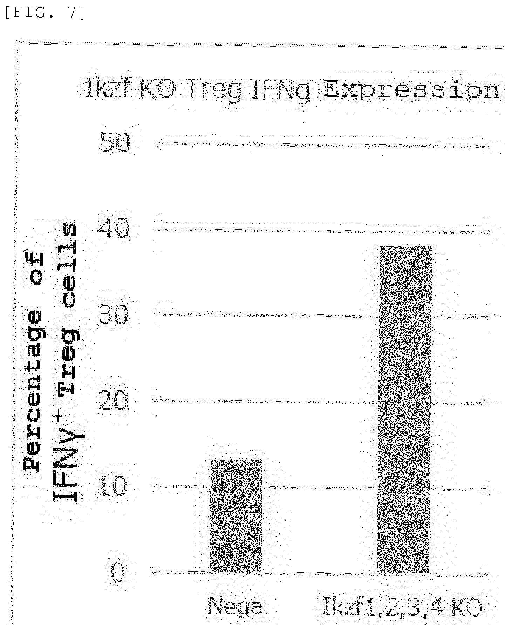

The results are shown in Figure 7. The results here show that knockout of all Ikzf1, 2, 3 and 4 also induced the change to IFN-γ-positive Treg cells in human Treg cells.

### Example 3-2: Change to IFN-γ-positive Treg cells upon treatment with Ikzf family degraders in human Treg cells

EasySep Human CD4+ T Cell Isolation Kit (STEMCELL) was used to human CD4⁺ cells. The cells were then stained with anti-CD4, anti-CD25, and anti-CD45RA antibodies, and Fr I (CD45RA⁺CD25⁺) and Fr II (CD45RA⁻CD25⁺⁺) were sorted using MA900 (SONY). After confirming the purity of the sorted samples, they were seeded in the presence of Dynabeads T-activator (2.5 µl/well, Thermo Fisher Scientific), IL-2 (100 u/ml, Miltenyi Biotec) and Pomalidomide (Ikzf1,3 degrader) or REF001329 (Ikzf2,4 degrader) at 1 × 10⁴ cells/well in 96-well plates. The cells and cultured for 9 days. Then, the supernatant was removed and Brefeldin A and Monensin, and Cell Activation Cocktail (Biolegend) were added thereto. After 5 hours of incubation, the cells were stained with antibodies against CD4 and CD25, fixed, and stained for IFN-γ and a nuclear proteins Foxp3, Ikzf1, and Ikzf2. Then, this samples were analyzed on CytoFLEX LX (Beckman Coulter).

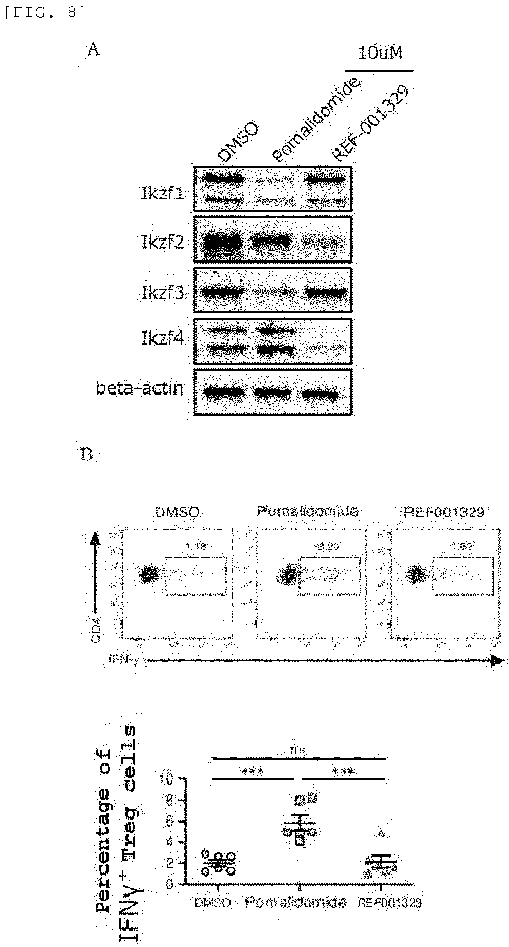

The results are shown in Figure 8. Figure 8A shows that Ikzf1,3 was decomposed by Pomalidomide and Ikzf2,4 was decomposed by REF001329. Figure 8B shows the percentages of IFN-γ positive Treg cells gated by flow cytometry. These results suggests that knockdown of Ikzf1,3 induced the change of Treg cells to IFN-γ-positive Treg cells. On the other hand, knockdown of Ikzf2,4 did not induce any change of Treg cells to IFN-γ-positive Treg cells.

Examples 3-1 and 3-2 indicated that Ikzf1 was also responsible for the change to IFN-γ-positive Treg cells in humans, suggesting that Ikzf1ΔE5 expression should be effective in humans similarly in mice, in view of Examples 2-1 and 2-2.

### Example 4

### Effect of treatment with Ikzf family degraders on induction of iTreg cells from naïve T cells in humans

Naive T cells were prepared from human PBMCs using Naive CD4+ T Cell Isolation Kit II, human (Miltenyi Biotec), and seeded onto plates coated with anti-CD3 antibody (8 µg/ml) in medium containing TGF-β (10 ng/ml) and IL-2 (200 U/ml). Then, various concentrations of Pomalidomide or REF001329 were added thereto and the cells were incubated for 10 days with medium changes and passaging. On days 4, 6, and 10 after the seeding, the cells were collected, and stained with Human Regulatory T Cell Sorting Cocktail (BD) and anti-Foxp3 antibody, followed by analyzing the differentiated iTreg cells with CytoFLEX LX (Beckman Coulter). An aliquot of the day 6 samples was evaluated for the protein amount of Ikzf family by Western blotting.

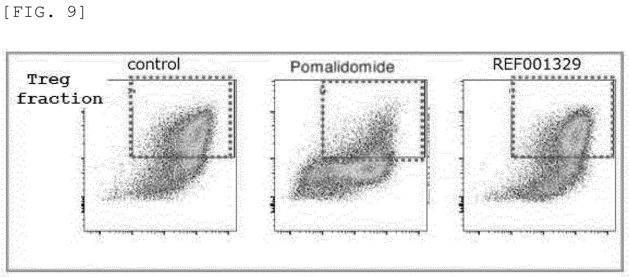

The results are shown in Figure 9. Figure 9 shows that, among the knockdowns of the Ikzf family, the knockdown of Ikzf1,3 with Pomalidomide suppressed the differentiation into iTreg cells. These results suggest that Ikzf1ΔE5 expression can also act on naive T cells and affect their differentiation into iTreg cells, in view of Examples 2.

### Example 5

### Investigation of anti-tumor effects of Ikzf1ΔE5 Treg cells in cancer-bearing mouse model

Foxp3eGFP-Cre-ERT2 and Foxp3eGFP-Cre-ERT2IkE5^{f/f} mice were subcutaneously implanted with melanoma cell line B16F0 or colon cancer cell line MC38, and tamoxifen was administered intraperitoneally on days 0, 1 and 3 after the implantation. Tumor diameter was measured every 2 days. On day 18, the tumors and the draining Lymph Nodes (dLNs), and non-draining lymph nodes (ndLNs) were sampled. Tumor-infiltrating lymphocytes (TILs) were prepared using Tumor dissociation kit and gentleMACS OCTO Dissociator according to the procedure manual.

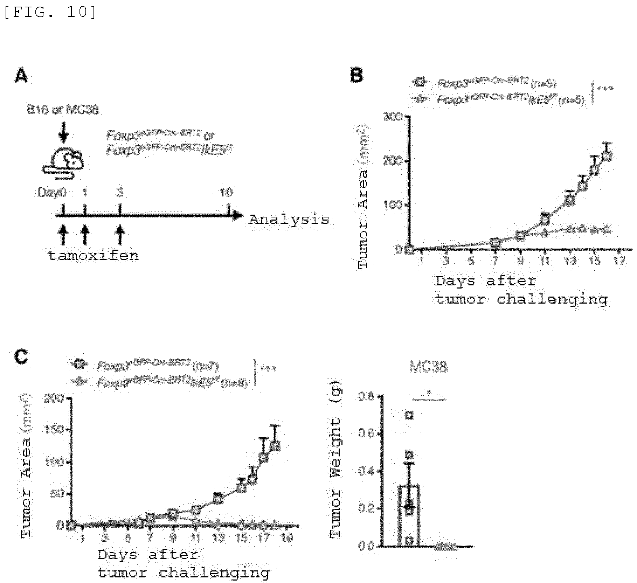

The results are shown in Figure 10. Figure 10A depicts the procedure in this Example. Figure 10B shows that the engraftment and the proliferation of B16FO cells were suppressed in Foxp3eGFP-Cre-ERT2IkE5^{f/f} mice expressing Ikzf1ΔE5, compared to Foxp3eGFP-Cre-ERT2. Figure 10C shows that the engraftment of MC38 cells was suppressed in Foxp3eGFP-Cre-ERT2IkE5^{f/f} mice expressing Ikzf1ΔE5, compared to Foxp3eGFP-Cre-ERT2. These results here suggest that Ikzf1ΔE5 may strongly suppress the engraftment and the proliferation of tumors, and in other word that Ikzf1ΔE5 can be used to prevent and/or treat tumors.

### Example 6

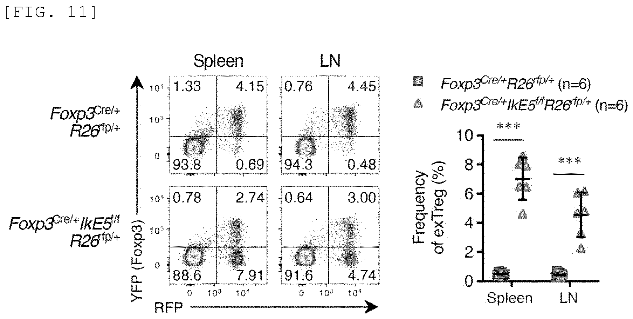

In order to evaluate the effect of Exon5 deletion in Ikzf1 on the stability of Treg cells in vivo, Foxp3^{Cre}IkE5^{f/f} mice were crossed with Rosa26^{RFP} reporter mice to create fate-mapping mice of Foxp3-expressing cells (Foxp3^{Cre/+}IkE5^{f/ f}R26^{RFP/+}). Rosa26^{RFP} reporter mice are genetically modified mice in which a STOP cassette flanked by loxP sites is inserted at the ROSA26 locus in front of the DNA sequence encoding Red Fluorescent Protein (RFP), and express RFP via removal of the STOP cassette upon Cre expression. Thus, FACS analysis for Foxp3^{Cre/+}IkE5^{f/ f}R26^{RFP/+} allow to detect Foxp3-expressing Treg cells as both RFP- and YFP-positive cell population, whereas T cells that once have expressed Foxp3 as Tregs and then lost Foxp3 expression (hereinafter may be referred to as "exTreg" or "exTregs") as RFP-positive and YFP-negative cell population. Therefore, according to this system, spleens and lymph nodes were collected from Foxp3^{Cre/+}R26^{RFP/+} and Foxp3^{Cre/+}IkE5^{f/f}R26^{RFP/+} mice (3-4 weeks old) and FACS analysis was used to measure the percentage of exTregs, showing that the percentage of exTregs was significantly increased in Foxp3^{Cre/+}IkE5^{f/f}R26^{RFP/+} mice (Figure 11). This suggests that Exon5 deletion in Ikzf1 impairs the stability of Tregs and induces the change of Tregs to exTregs.

### Example 7

### Analysis for the expressions of target genes of p300 and NFAT1 in Ikzf1ΔE5-expressing Treg cells

CD4+FYP+ Treg cells purified from the spleen and lymph nodes of the Foxp3^{Cre}IkE5^{f/f} mice were stimulated with Cell Stimulation Cocktail (eBioscience) for 1 hour at 37°C. The stimulated Treg cells were then treated with 1% formaldehyde for 30 minutes at room temperature. After extracting the nucleus, the DNAs were fragmented by sonication using a Digital Sonifier (Branson) and they were incubated overnight at 4°C with 5 µg of anti-Foxp3 antibody (Abcam), anti-p300 antibody (Abcam) or anti-NFAT1 antibody (Abcam) that was previously reacted with 100 µl DynaBeads IgG magnetic beads (Thermo Fisher Scientific). After the incubation, the DynaBeads were washed 4 times with RIPA buffer (1 ml/sample), and once with TE-NaCl buffer (1 ml/sample), and the DNAs were extracted with elution buffer (100 µl/sample). After the extraction, DNAs were reverse cross-linked at 65°C, 1100 rpm for 24 hours and purified using a ChIP DNA Clean & Concentrator (Zymo Research) (40 µl/sample). The purified ChIP DNAs were fragmented using Covaris Focused-ultrasonicator S220 (Covaris) before library preparation. Libraries were prepared using the Ion Xpress Plus Fragment Library Kit (thermo Fisher Scientific) according to the manufacturer's instructions, and sequenced using the IonS5 sequencer system (thermo Fisher Scientific). As a control, Treg cells similarly obtained from Foxp3^{Cre} mice were used to quantify the change in p300 and NFAT1 genomic binding around the enhanced Foxp3-binding genomic sites by inducing the Ikzf1ΔE5 expression, thereby comparing both results.

For data analysis, the quality of the sequencing reads was first checked using fastQC to confirm that the average Phred score was 20 or over. Then, the sequencing reads were trimmed with fastx_trimmer in Fastx_tool kit and the sequencing reads were mapped to mouse genome mm10 using Bowtie2 with default settings. Peakcall was performed using MACS2. ChIP-seq peaks were defined by FindPeaks, in which size was set to 500 base pairs, minimum distance between peaks was set to 500 base pairs, FDR was set to default, and local filter was set to OFF. Regions where Foxp3 binding is common between wild-type Treg cells and Ikzf1ΔE5-expressing Treg cells and regions unique to each cell were identified using HOMER (getDifferentialPeaks) with default parameters. Normalized density plots of p300 and NFAT1 binding peaks around the Foxp3 binding site were calculated using annotatePeaks in Homer package.

The resulting normalized density plots of p300 and NFAT1 binding peaks around the Enhanced Foxp3-binding Sites are shown in Figure 12. This shows that the histone acetyltransferase (HAT), p300, interacted with Foxp3 and the binding to the Enhanced Foxp3-binding Sites was enhanced in Ikzf1ΔE5-expressing Treg cells, with also taking account of Figure 4. These results indicate that Foxp3 forms a negative complex with Ikzf1 and may compete with the active Foxp3-p300 complex in the control of gene transcriptions. Figure 12 shows that the binding of NFAT1 to the Enhanced Foxp3-binding Sites was indeed increased in Ikzf1ΔE5 Treg cells, suggesting that the binding of NFAT1 to the Enhanced Foxp3-binding Sites would be inhibited. Thus, it was revealed that the Foxp3-Ikzf1 complex competes with co-activators such as p300 and NFAT1 by inducing a closed chromatin structure via an Ikzf1-dependent nucleosome reorganization deacetylase (NuRD) complex, resulting in suppression of its target gene expressions.

### INDUSTRIAL APPLICABILITY

The present invention provides technology related to new methods for treatment and prevention of tumors and other diseases, and has great industrial applicability.

## Claims

1. A composition for preventing and/or treating tumors, which comprises an Ikzf1ΔE5 protein or an Ikzf1ΔE5 gene-related substance.

2. The composition for preventing and/or treating tumors according to claim 1, wherein the composition targets a T-cell.

3. The composition for preventing and/or treating tumors according to claim 2, wherein the T-cell is a CD4-positive T-cell.

4. The composition for preventing and/or treating tumors according to claim 2, wherein the T-cell is a regulatory T-cell and/or a naive T-cell.

5. The composition for preventing and/or treating tumors according to any one of claim 1 to 4, wherein the Ikzf1ΔE5 gene-related substance is a nucleic acid molecule encoding the Ikzf1ΔE5 protein.

6. The composition for preventing and/or treating tumors according to any one of claim 1 to 4, wherein the Ikzf1ΔE5 gene-related substance is a substance that deletes part or all of the exon 5 portion of Ikzf1 gene.

7. The composition for preventing and/or treating tumors according to claim 6, wherein the substance that deletes part or all of the exon 5 portion of Ikzf1 gene is selected from the group consisting of exon skipping agents for exon 5 of Ikzf1 gene, and gene therapy vectors such as CRISPR/Cas.

8. The composition for preventing and/or treating tumors according to claim 7, wherein the exon skipping agent is a nucleic acid.

9. The composition for preventing and/or treating tumors according to claim 1, which comprises a T cell expressing Ikzf1ΔE5.

10. A method for screening a substance for modulating the formation of an Ikzf1 complex containing Ikzf1 protein, and Foxp3 protein, CHD4 protein and/or HDAC1 protein, which comprises the steps of:
(a) contacting a system comprising Ikzf1 protein or a fragment thereof, and Foxp3 protein or a fragment thereof, CHD4 protein or a fragment thereof and/or HDAC1 protein or a fragment thereof with a candidate compound of a substance for modulating the formation of an Ikzf1 complex,
(b) determining whether the candidate compound inhibits or promotes the association of Ikzf1 protein or a fragment thereof with Foxp3 protein or a fragment thereof, inhibits or promotes the association of Ikzf1 protein or a fragment thereof with CHD4 protein or a fragment thereof, inhibits or promotes the association of Ikzf1 protein or a fragment thereof with HDAC1 protein or a fragment thereof, inhibits or promotes the association of Foxp3 protein or a fragment thereof with CHD4 protein or a fragment thereof, and/or inhibits or promotes the association of Foxp3 protein or a fragment thereof with HDAC1 protein or a fragment thereof, and
(c) identifying the candidate compound as a substance for modulating the formation of an Ikzf1 complex when the compound inhibits or promotes any one of the associations.

11. The method according to claim 10, wherein the substance for modulating the formation of an Ikzf1 complex is a substance for inhibiting or promoting the association of Ikzf1 protein or a fragment thereof with Foxp3 protein or a fragment thereof.

12. The method according to claim 10 or 11, wherein a substance for modulating the formation of an Ikzf1 complex is a composition for preventing or treating tumors, a composition for modulating interferon gamma production, or a composition for tailoring T cells.
